# EUROPEAN PATENT SPECIFICATION

(11) **EP 1 242 147 B1**
(45) Date of publication and mention of the grant of the patent: **03.01.2007**
(21) Application number: 00987897.6
(22) Date of filing: 12.12.2000
(51) Int. Cl.: A61N 1/372

(54) **PROGRAMMING SYSTEM FOR MEDICAL DEVICES**
PROGRAMMIERSYSTEM FÜR MEDIZINISCHE GERÄTE
SYSTEME DE PROGRAMMATION POUR DISPOSITIFS MEDICAUX

(30) Priority: 16.12.1999 SE 9904627
(43) Date of publication of application: 25.09.2002
(73) Proprietor: St. Jude Medical AB, 175 84 Järfälla (SE)
(72) Inventor: SAMUELSSON, Eric, S-177 53 Järfälla (SE); ANDERSSON, Jonas, S-121 38 Johanneshov (SE)
(86) International application number: PCT/SE2000/002520
(87) International publication number: WO 2001/043822

(56) References cited:
- EP-A2- 0 773 038
- US-A- 5 800 473
- US-A- 5 833 623

## Description

### Field of Invention

The invention relates generally to implantable medical devices and more particularly to systems for programming implantable medical devices.

### Background Art

Implantable medical devices perform multiple highly complex functions which may be adapted to the particular conditions and needs of specific patients. For example implantable cardiac pacers, which provide stimulating impulses to a heart with a disturbed cardiac rhythm, can be configured with various different parameters values and functions depending on the particular condition of a patient. By way of example, a cardiac pacer that is presently on the market allows in excess of 40 different parameters to be programmed.

In addition to the programmable parameters, a cardiac pacer generally stores a large quantity of measured data. Conventional pacers are commonly equipped with sensors for monitoring the activity of the heart. Information obtained through monitoring can be used for diagnosing certain patient conditions, which in turn can be addressed by adapting the pacer functions in some way. The programming and interrogation of implanted devices is commonly performed non-invasively using a computer- or microprocessor-based programmer, which communicates with the pacer via a telemetry link. These programmers include a display and some form of keyboard, which may be implemented as a touch sensitive screen, for the input of data. When the programmer interrogates the implanted device, stored and measured data will be transferred to the programmer.

This information must be displayed to the operator. Parameter values, whether programmable, measured or fixed, are displayed as numerical or alphanumerical values. An example of such a programmer is described in US 5,833,623. The manner in which this information is organised for display varies from programmer to programmer. US 5,713,937 describes a programmer where characteristics in a graph illustrating an ECG can be added to the graph by selecting and dragging icons from a menu bar to the graph. However, conventional programmers typically display programmable parameters and measured data separately. Moreover, with the large amount of data provided by present day pacers, the programmable information in many cases is further divided into subgroups for display. For example, programmable parameters may include basic parameters, extended parameters, sensor parameters and patient data. Such groupings are typically chosen for technical reasons related to the internal organisation of the programmer. For example some parameters may require additional interrogation of the pacer, while others may be more readily available. Consequently, an operator of a programmer must be very familiar with the programmer organisation if he is to operate the programmer effectively and to full effect.

The majority of such medical devices are programmed and monitored by medically skilled practitioners, who have a thorough understanding of the patient's condition, but may have less knowledge of the possibilities of the programmer and/or the medical device. Moreover, they may be required to monitor several different types of medical devices, working in different modes and implanted in patients with different diagnoses. As a result of this, an operator may encounter two medical devices of the same type and configured in the same way for the same patient diagnosis only infrequently. Consequently, there is a need for a programmer that is simple to operate and which can be used intuitively.

It is thus an object of the invention to provide a programmer for monitoring and controlling the operation of an implantable device that is easy to operate, and thus enables an operator to exploit all possible functions of a medical device with little knowledge of the programmer.

It is thus an object of the invention to provide a programmer that provides the operator with an interface to the medical device that is easy to understand and operate, and thus enables an operator to exploit all possible functions of a medical device with little knowledge of the programmer.

### SUMMARY OF INVENTION

This object is achieved by an arrangement for monitoring and controlling the operation of an implantable medical device, a method, a computer program product, and a computer readable storage medium as defined by the independent claims.

By linking parameters to a graphical representation of a quantity influenced by the operation of said medical device, the operator will be able to select parameters on the basis of the effect intended, rather than setting parameters and then observing the effect. The resulting system is thus not only more intuitive and thus easier to use for a medical practitioner, it will also be safer for the patient, since there is less likelihood of the operator programming unsuitable parameter values.

Preferably the quantity is a measurable physiological activity commonly used for evaluating the condition of the patient. For example, when the medical device is a cardiac stimulating device, a useful quantity for use in programming the device control parameters is the electrical activity of the heart in the form of an electrocardiogram (ECG) or intracardial electrogram (IEGM), which is commonly measured by the device or the programmer and serves as an important diagnostic tool for evaluating the condition of the patient and the operation of the implanted device. Other waveforms, for example showing the variation of the quantity over time, are also useful for programming parameter values.

Obviously, some implanted devices will only permit specific discrete parameter values to be used. The programming arrangement thus advantageously includes means for setting a nearest authorised modified parameter value in response to the altered shape of the graphical representation.

The graphical representation may be a stored representation of the quantity. Advantageously, however, the arrangement includes means for collecting data relating to said quantity, and means for constructing a graphical representation of said quantity for display. In this way any desired effect expressed by that manipulation of the graphical representation will be more closely related to the actual effect achieved after modification of the parameters.

Preferably, several parameters are associated with portions of the same graphical representation, such that any alteration in shape of the graphical representation causes the modification of all parameters at the same time. This not only saves time, but also enables the illustration of any naturally existing interdependence between parameters, which results in a more intuitive and understandable procedure for a clinician.

### BRIEF DESCRIPTION OF THE DRAWINGS

Further objects and advantages of the present invention will become apparent from the following description of the preferred embodiments that are given by way of example with reference to the accompanying drawings. In the figures:
- Fig. 1: schematically shows a system for programming a medical device in accordance with the present invention,
- Fig. 2: shows a window of the graphical user interface for programming AV delay and base rate,
- Fig. 3: shows a window of the graphical user interface for programming capture threshold and evoked response sensitivity,
- Fig. 4: shows a screen of the graphical user interface for programming parameter values according to a further embodiment of the invention,
- Fig. 5: shows a variation of the embodiment of Fig. 3

### DETAILED DESCRIPTION OF THE DRAWINGS

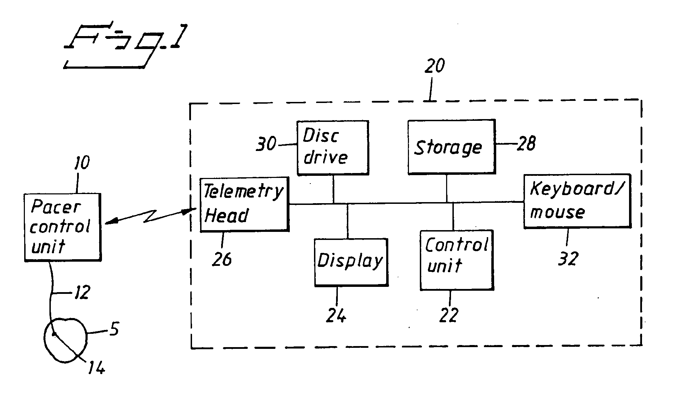

Fig. 1 schematically shows an arrangement for programming an implanted medical device. In the illustrated embodiment, the medical device is a cardiac pacer implanted in a patient. The pacer comprises a pacer control unit 10, which is generally implanted pectorally near the shoulder of the patient under the skin and one or more electrodes 14, which are anchored in the patient's heart 5. One or more leads 12 connect the electrodes 14 to the pacer control unit 10. The pacer is capable of operating autonomously and is powered by a battery (not shown). The electrodes 14 are used to apply stimulating pulses to the heart tissue and may also sense the electrical activity of the heart and possibly other physiological activities, such as respiration. This information is stored in the pacer control unit 10, for example in the form of an intracardial electrogram (IEGM) for later consultation by a medical practitioner during a routine check. Other information collected and stored in the pacer control unit 10 can include data relating to the condition of the pacer, such as the residual battery power or the impedance of the leads 12. The information stored in a pacer can be consulted using a programmer 20 which is preferably computer- or microprocessor based.

The programmer 20 includes a control unit 22, a display 24, a telemetry head 26, internal storage means 28 and some form of data input device 32 that may be a keyboard, a mouse, a touch-sensitive screen, or the like, or some combination of these. A disk drive 30 may also be provided in the programmer 20 for receiving a diskette, CD ROM or similar portable storage element capable of carrying computer-readable code. Software used for controlling the operation of the programmer is stored in the storage means 28 and executed by the control unit 22 using the storage means 28. Additional software applications can be provided on a removable diskette and read with the aid of the disk drive 32. These additional software applications may be used to optionally extend the functions of the programmer. Alternatively, additional software applications may assure more basic functions that are specific to a class of implantable device; this may be of interest when the internal storage means 28 of the programmer 20 are of limited capacity, for example. Communication between the programmer 20 and the pacer control unit 10 is effected via a telemetry link, whereby the telemetry head 26, which preferably includes an inductive coil, is placed over the implantation site of the pacer control unit 10. Once a link has been established, the programmer 20 interrogates the pacer control unit 10 and downloads the stored information. Modifications to the pacer settings programmed by the programmer 20 are also uploaded to the pacer control unit via the telemetry link. Exchanges of information between the pacer control unit 10 and the programmer 20 may occur throughout a programming session, for example to obtain recent ECG or IEGM signals recorded by the pacer 10 when testing a modified parameter value.

Interaction between the operator and the programmer 20 is through the display 24 and input device 32 and more particularly through a graphical user interface of the programmer 20. Through the graphical user interface, the operator may use the programmer 20 to set programmable parameter values, carry out tests on the implantable device and also view diagnostic data.

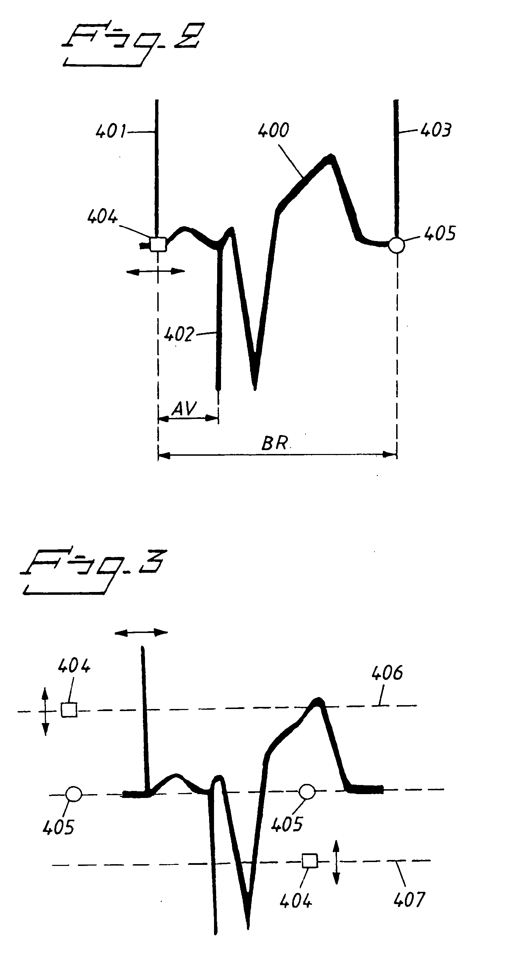

In accordance with the present invention, the programmer 20 displays parameter values in graphical form. More specifically, the programmer 20 displays a representation of a quantity that is influenced by the operation of the implanted medical device. Preferably this quantity is also measured by the implanted medical device. Preferably the quantity represents a physiological activity influenced by the device and commonly used by a clinician for evaluating the patient's condition and also the operation of the implanted device 10. The parameters used for controlling the operation of the medical device are mapped to this representation in such a way that a variation in the shape of the representation causes the programmer to effect a corresponding variation in the value of the mapped parameters. The representation of a physiological activity is preferably in the form of a waveform. In the present example which relates to a cardiac pacer 10, the waveform is an ECG or IEGM, which is routinely measured during programmer sessions. Data representing the measured electrical activity of the heart in the form of an ECG or IEGM is also readily available from the pacer device itself. By displaying parameters in such a graphical form, and moreover, permitting the modification of parameters by manipulating the graphical representation, the operator knows immediately what effect the programmed parameters will have on the patient's condition. This is described in more detail with reference to Fig. 2.

Fig. 2 shows a parameter programming window of the graphical user interface. It is assumed that this window is displayed by the programmer following the selection by the operator of the programmable parameter in a graphical user interface of the programmer 20. The parameter selected is the base rate (BR) of the pacer 10.

The waveform 400 depicted in this window represents one cycle of an ECG in which atrial 401 and ventricular 402 stimulation pulses are shown. The atrial stimulation pulse 403 for the following cycle is also shown in the waveform. For this waveform, the programmable parameter for the basic interval BR of the pacer is mapped to the distance between the two atrial stimulation pulses 401, 403. The basic interval is the period between two consecutive paced events without intervening intrinsic activity. While the basic interval is not a common programmable parameter for most pacers, it is inversely proportional to the base rate, which is commonly used. The programmer may associate either base rate values or basic interval values with this distance. A further parameter, namely the AV delay, AV, is also mapped to a portion of the waveform, specifically to the distance between the first atrial stimulation pulse 401 and the ventricular stimulation pulse 402. Select points on the curve are defined as movable or fixed. In Fig. 2, the starting point 404 represented by a square is a movable point, while the end point 405 represented by a circle is a fixed point. By selecting and dragging the movable point 404, either using a cursor control device, such as a mouse, or by keyboard or keypad controls, the operator can alter the length of the curve 400, and specifically the mapped portions of the curve. Since both the basic interval BR and the AV delay AV depend on distances measured in parallel to the horizontal axis of the curve 400, the movable point 404 is constrained to move along a straight line defined by the axis (the time axis) of the curve 400.

The programmer 20, and specifically the control unit 22 automatically adjusts the parameter values linked to the dimensions of the curve 400. For example if the operator moves the movable point to the left in Fig. 2 to lengthen the curve, the basic interval will be lengthened and the base rate reduced accordingly. Similarly, a reduction in the curve length by moving the movable point to the right in the figure will reduce the basic interval. Once an operator has made the desired adjustment to the curve, he may program the pacer device 10 with the associated parameter values. This is preferably achieved by providing a separate "program" command button on the screen. On registering this command, the control unit 22 of the programmer 20 sends the new parameter values to the pacer device 10 through the telemetry head 26. Only basic interval or base rate values allowed by the pacer device will be programmed. Thus if the pacer permits specific discrete values of base rate only, the programmer will set the permitted value that is closest to that defined by the modified curve. In the same way, the upper and lower limits of any parameter value is similarly observed. The upper and lower limits of a parameter are preferably also illustrated graphically by preventing further movement of the curve when a limit has been reached. In addition, the two end points of any parameter value may be illustrated graphically as limits to the curve variation to aid the operator in scaling any adjustment.

When the movable point 404 of the curve is shifted, this causes a stretching of the curve throughout its length. Thus all the intervals defined along its length will be modified in scale with the increase in length. In other words, the curve 400 retains its relative proportions in the direction of movement in analogy with normal physiological processes. Thus in the curve depicted in Fig. 2, an increase in the basic interval BR or reduction of the base rate caused by the extension of the curve width will automatically cause an in-scale variation of the AV delay (interval). When the operator programs a modified base rate, the control unit 22 will automatically send the adjusted AV delay value to the pacer 10. Other parameters that are likewise affected by a variation in the basic interval, such as the PV delay or the refractory period, could also be associated with the curve. This permits different parameters to be altered and programmed using the same curve. For example supposing that at a base rate of 90 pulses per minute (ppm), the AV interval,AV,must not exceed 300ms. The restriction in the value of AV delay ,AV,may be automatically implemented by the programmer when the operator adjusts the base rate by altering the total length of the curve 400.

Different parameters may also be programmed independently using the same curve. For the programming of each separate parameter, a different set of fixed 405 and movable points 404 are displayed. The different programmable parameters may be selected by the operator using an appropriate command from a menu bar incorporated in the parameter programming window. Alternatively, or in addition, the desired parameter may be selected by selecting the appropriate portion of the curve 400 to make movable 404 and fixed points 405 appear.

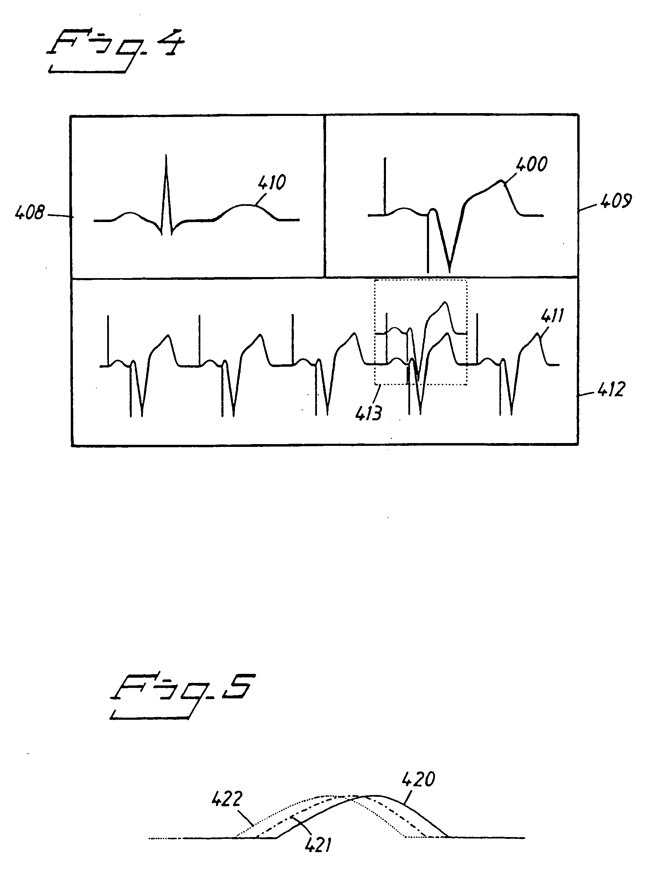

The representation of parameters in terms of their effect on a physiological activity as illustrated in Fig. 2 is preferably also utilised for the depiction of diagnostic tests and measured data. This is shown by way of example in Fig. 3 which shows a further screen of the graphical user interface. Here the same curve 400 is depicted as in Fig. 2. Two threshold levels 406, 407 are shown in the form of horizontal lines. The uppermost line 406 in Fig. 3 represents the atrial capture threshold and the lower line 407 represents the evoked response (ER) sensitivity threshold, which relates to ventricular stimulation. Fixed points 405 are located on the central horizontal axis of the curve 400. Movable points 404 are located on the lines 406, 407. These lines 406, 407 are thus movable with respect to the central (horizontal) axis of the curve, whereby an increase in the distance between a threshold line and the central axis causes an increase in threshold value, and a decrease in distance results in a decrease in threshold value. In the illustrated example, the evoked response sensitivity threshold value 407 can be viewed or set by moving the line. The atrial capture threshold is preferably used to indirectly adjust the atrial pulse width, and so adjust the atrial pulse energy. Reducing the threshold level by moving the line 406 towards the central axis of the curve 400 results in an increase in the atrial pulse width, while an increase in the threshold level 406 causes a reduction in atrial pulse width.

For the example of a cardiac pacer described with reference to Figs. 2 and 3, it is possible to represent substantially all programmable parameters and illustrate all tests utilising waveforms which are graphical representations of a ECG cycle showing pacer stimulation pulsed and a ECG waveform of spontaneous activity.

As mentioned above, the waveform used to represent programmable parameters of an implanted medical device 10 may be derived from data collected by the device 10 itself or even from the programmer 20. Thus, for example, a programmer 20 of an implantable cardiac pacer 10 may utilise ECG or IEGM curves which are routinely recorded by the pacer 10 and sometimes by programmer 20 during follow-up visits by the patient at a clinic or surgery to depict the parameters. This brings with it the possibility of utilising a waveform that is generated from the recorded data, to provide a real picture of the effects of programming parameters. This possibility is utilised in a further embodiment of the invention using a parameter programming screen as depicted in Fig. 4. The screen in Fig. 4 is divided into an upper and a lower portion, with two windows 408, 409 in the upper portion and a single window 412 comprised in the lower portion. The two upper windows 408, 409 show waveforms used for programming parameters. The waveform 400 representing an ECG cycle showing atrial and ventricular stimulation is depicted in the right-hand upper window 409 and a waveform 410 representing a spontaneous ECG cycle 410 is depicted in the left-hand upper window 408. As mentioned above, these two waveforms 400, 410 can be used to program substantially all programmable parameters of a cardiac pacer, as well as illustrating tests and diagnostics. In the third window 412 depicted in the lower half of the screen there is displayed a continuously rolling waveform 411 constructed from data recorded by the pacer device or the programmer. The operator changes the shape of the waveforms 400, 410 in the two uppermost windows 408, 409 to program parameters and can observe the result on the recorded waveform 411 depicted in the lower window 412. In order to compare the results between the desired programmed values input via the waveforms 400 or 410 and the resulting measured waveform 411, the programming waveforms 400 and 410 may be copied and superimposed on parts of the measured waveform 411. This is illustrated in the lower window 412 of Fig. 4. Preferably this is implemented using a drag and drop function so that the operator may simply select the programming waveform 400, 410 and drag this down to the measured waveform 412 for comparison. The displaced waveform is automatically modified to the scale of the measured waveform 412 to enable a useful comparison. A freeze function is associated with the recorded waveform 411 and may be invoked to facilitate this comparison.

The waveforms 400 and 410 may be stylised waveforms stored in the programmer before use. Alternatively, the waveforms 400 and 410 may be generated from data recorded by the pacer device 10 or the programmer 20 some time previously or during programming of the cardiac pacer 10. This may be achieved in different ways. For example, when the programmer 20 is started, the initial waveforms 400, 410 displayed in the uppermost windows 408 and 409 will be simulated waveforms that are stored in the programmer 20. As a follow-up procedure progresses and measured data is obtained from the cardiac device 10 or the programmer 20, the waveforms 400 and 410 are replaced by a waveform based on the measured data. For example, the stimulated ventricular pulse amplitude may be measured by the pacer output stage. This values combined with the IEGM measured by the pacer 10 can then be combined to form a waveform 400 for programming. Since the measured ECG or IEGM waveform will vary slightly from cycle to cycle, the displayed waveforms 400 and 410 may be generated using the averaged data for several measured ECG or IEGM cycles.

In a further embodiment of the invention, the effects of varying the parameters as described with reference to Figs. 3 and 4 is not shown using a continuously varying waveform as depicted in the lower window of Fig. 4. Rather the most recently available single waveform cycle is depicted with a number of earlier cycles shown offset from the most recent cycle. This is illustrated in Fig. 5. A current waveform 420 is depicted by a continuous line, earlier cycles 421 and 422 are depicted with dashed lines. In addition to being offset, earlier cycles are preferably also depicted in a contrasting shade or colour as illustrated by the different broken lines in Fig. 5. This provides the operator with immediate feedback of the effects of altering one or more parameters.

In the exemplary embodiment using a programmer for a cardiac pacer, the parameters are linked to a representation of an ECG or IEGM signal. It will be appreciated that a measured or simulated ECG or IEGM waveform could equally be used to control intracardial devices (ICD) and devices for emerging indications (EI), for instance. In the case of devices for emerging indications (EI), which stimulate both sides of the heart, IEGM curves representing or measured from different sides of the heart can be compared and manipulated relative to one another to modify parameters that control the timing between the sides of the heart. It will be understood that the invention is not limited to ECG or IEGM waveforms. Any quantity that may be represented graphically and that is influenced by or influences the operation of an implantable medical device can be used for programming the device. The choice of quantity depends on the workings of the device and the types of parameters to be programmed. For example programmers for drug pumps may utilise a representation of the heart rate or respiration rate, neurostimulators may utilise waveforms representing electrical activity in the brain.

## Claims

1. An arrangement for monitoring and controlling the operation of an implantable medical device, comprising means (26, 10) for communicating with said medical device (10), means (22, 24) adapted to display a graphical representation in the form of a curve or waveform (400) of a quantity influenced by the operation of said medical device,
means (22) adapted to associate parameters relating to the control of the device with portions of said graphical representation,
means (32, 22) adapted to select and move points in said graphical representation to thereby alter the shape of said displayed graphical representation to illustrate a desired operational effect of said medical device on said quantity, and
means (22, 24) responsive to said altered shape adapted to modify the values of associated parameters in accordance with the modification of the portions.

2. An arrangement as claimed in claim 1, **characterised in that** said quantity is at least one physiological activity influenced by said medical device.

3. An arrangement as claimed in claim 1 or 2, **characterised by** means adapted to set a nearest authorised modified parameter value in response to said altered shape of the graphical representation.

4. An arrangement as claimed in any one of claims 1 to 3, **characterised in that** said waveform is a representation of the variation of said quantity against time.

5. An arrangement as claimed in any previous claim, **characterised by** means (26, 10) adapted to collect data relating to said quantity, and means (22) construct a graphical representation of said quantity for display.

6. An arrangement as claimed in any previous claim, **characterised in that** several parameters are associated with portions of the same graphical representation, such that said means responsive to the altered shape of a graphical representation are adapted to modify the values of said several parameters in accordance with modified portions.

7. An arrangement as claimed in any previous claim, **characterised in that** the graphical representation is a waveform representing the electrical activity of a patient's cardiovascular system.

8. An arrangement as claimed in any previous claim, wherein said medical device is a cardiac stimulating device.

9. A method for monitoring and/or programming the operation of an implantable medical device, **characterised by**:
displaying a graphical representation in the form of a curve or waveform (400) of a quantity influenced by the operation of said medical device,
mapping parameters relating to the control of the device onto select portions of said graphical representation,
altering the shape of said graphical representation to display a desired operational effect of said medical device on said quantity, and
programming the associated control parameter in response to said altered shape.

10. A method as claimed in claim 9, **characterised in that** said quantity is at least one physiological activity.

11. A method as claimed in claim 9 or 10, **characterised in that** said mapping step includes mapping parameter magnitudes with selected dimensions of said graphical representation, and said modifying step includes modifying the magnitude of the control parameter in response to a change in said dimensions.

12. A method as claimed in any one of claims 9 to 11, **characterised in that** said graphical representation is a waveform representing the variation of said quantity against time.

13. A method as claimed in any one of claims 9 to 12, **characterised by** generating said graphical representation from a plurality of measured curves and/or values.

14. A method as claimed in any one of claims 9 to 13, **characterised by** mapping several parameters to one graphical representation, and modifying the several parameters in response to the altering of shape of the graphical representation.

15. A method as claimed in any one of claims 9 to 14, **characterised in that** said display step includes displaying a curve representing at least one cycle of an electrocardiogram (ECG) or intracardial electrogram (IEGM) signal.

16. A method as claimed in any one of claims 9 to 15, **characterised by** displaying a measured signal of the quantity for comparison with said graphical representation.

17. A method as claimed in claim 16, **characterised by** comparing said graphical representation with said signal by superposing said graphical representation on said displayed signal.

18. A method as claimed in claim 17, **characterised in that** said superposing step includes automatically adjusting said graphical representation to the scale of said measured signal.

19. A method as claimed in any one of claims 16 to 18, **characterised in that** said displayed measured signal varies continuously over time.

20. A method as claimed in claim 18, **characterised by** displaying offset and contrasting signal portions deriving from signal values measured in different time periods.

21. A computer program product, **characterised by** computer readable code, which when loaded in digital processing means causes the execution of the method as claimed in any one of claims 9 to 18.

22. A computer readable storage medium carrying a computer program product as claimed in claim 21.

## Patentansprüche

1. Anordnung zur Überwachung und Steuerung des Betriebs einer implantierbaren medizinischen Vorrichtung, enthaltend
eine Vorrichtung (26. 10) zum Kommunizieren mit der genannten medizinischen Vorrichtung (10),
eine Vorrichtung (22, 24), ausgelegt eine grafische Repräsentation in Form einer Kurve oder Wellenform (400) einer Größe anzuzeigen, die durch den Betrieb der genannten medizinischen Vorrichtung beeinflusst wird,
eine Vorrichtung (22), ausgelegt, Parameter, die sich auf die Steuerung der Vorrichtung beziehen, mit Teilen der genannten grafischen Repräsentation zu verknüpfen,
eine Vorrichtung (32, 22), ausgelegt in der genannten grafischen Repräsentation Punkte auszuwählen und zu bewegen, um hierdurch die Form der genannten angezeigten grafischen Repräsentation zu verändern, um eine gewünschte betriebliche Wirkung der genannten medizinischen Vorrichtung auf die genannte Größe darzustellen, und
eine auf die genannte geänderte Form reagierende Vorrichtung (22, 24), ausgelegt, die Werte der zugeordneten Parameter gemäß der Modifikation der Teile zu modifizieren.

2. Anordnung, wie in Anspruch 1 beansprucht, **dadurch gekennzeichnet, dass** die genannte Größe wenigstens eine durch die genannte medizinische Vorrichtung beeinflusste physiologische Aktivität ist.

3. Anordnung, wie in Anspruch 1 oder 2 beansprucht, **gekennzeichnet durch** eine Vorrichtung, ausgelegt, einen nächstliegenden, autorisierten modifizierten Parameterwert in Reaktion auf die genannte geänderte Form der grafischen Repräsentation einzustellen.

4. Anordnung, wie in einem der Ansprüche 1 bis 3 beansprucht, **dadurch gekennzeichnet, dass** die genannte Wellenform eine Repräsentation der Veränderung der genannten Größe gegenüber der Zeit ist.

5. Anordnung, wie in einem der vorhergehenden Ansprüche beansprucht, **gekennzeichnet durch** eine Vorrichtung (26, 10), ausgelegt um Daten, die sich auf die genannte Größe beziehen, zu sammeln und eine Vorrichtung (22), ausgelegt um eine grafische Repräsentation der genannten Größe für die Anzeige zu erstellen.

6. Anordnung, wie in einem der vorhergehenden Ansprüche beansprucht, **dadurch gekennzeichnet, dass** mehrere Parameter mit Teilen der selben grafischen Repräsentation so verknüpft sind, dass die genannte Vorrichtung, die auf die geänderte Form einer grafischen Repräsentation reagiert, in der Lage ist, die Werte der genannten verschiedenen Parameter in Übereinstimmung mit modifizierten Teilen zu modifizieren.

7. Anordnung, wie in einem der vorhergehenden Ansprüche beansprucht, **dadurch gekennzeichnet, dass** die grafische Repräsentation eine Wellenform ist, die die elektrische Aktivität des kardiovaskulären Systems eines Patienten repräsentiert.

8. Anordnung, wie in einem der vorhergehenden Ansprüche beansprucht, wobei die genannte medizinische Vorrichtung eine Herzstimulationsvorrichtung ist.

9. Verfahren zum Überwachen und/oder Programmieren des Betriebs einer implantierbaren medizinischen Vorrichtung, **gekennzeichnet durch**:
Anzeigen einer grafischen Repräsentation in Form einer Kurve oder Wellenform (400) einer **durch** den Betrieb der genannten medizinischen Vorrichtung beeinflussten Größe,
Abbilden von Parametern, die sich auf die Steuerung der Vorrichtung beziehen, auf ausgewählte Teile der genannten grafischen Repräsentation,
Ändern der Form der genannten grafischen Repräsentation, um eine gewünschte betriebliche Wirkung der genannten medizinischen Vorrichtung auf die genannte Größe anzuzeigen, und
Programmieren der zugeordneten Steuerparameter in Reaktion auf die genannte geänderte Form.

10. Verfahren, wie in Anspruch 9 beansprucht, **dadurch gekennzeichnet, dass** die genannte Größe wenigstens eine physiologische Aktivität ist.

11. Verfahren, wie in Anspruch 9 oder 10 beansprucht, **dadurch gekennzeichnet, dass** der genannte Abbildungsschritt das Abbilden von Parametergrößen mit ausgewählten Dimensionen der genannten grafischen Repräsentation umfasst und der genannte Modifizierungsschritt das Modifizieren der Größe der Steuerparameter in Reaktion auf die Änderung in den genannten Dimensionen umfasst.

12. Verfahren, wie in einem der Ansprüche 9 bis 11 beansprucht, **dadurch gekennzeichnet, dass** die genannte grafische Repräsentation eine Wellenform ist, die die Veränderung der genannten Größe gegenüber der Zeit darstellt.

13. Verfahren, wie in einem der Ansprüche 9 bis 12 beansprucht, **gekennzeichnet durch** Erzeugen der genannten grafischen Repräsentation aus einer Vielzahl von gemessenen Kurven und/oder Werten.

14. Verfahren, wie in einem der Ansprüche 9 bis 13 beansprucht, **gekennzeichnet durch** Abbilden mehrerer Parameter zu einer grafischen Repräsentation und Modifizieren der mehreren Parameter in Reaktion auf die Änderung der Form der grafischen Repräsentation.

15. Verfahren, wie in einem der Ansprüche 9 bis 14 beansprucht, **dadurch gekennzeichnet, dass** der genannte Anzeigeschritt das Anzeigen einer Kurve umfasst, die wenigstens einen Zyklus eines Elektrokardiogramm (ECG)- oder eines intrakardialen Elektrogramm (IEGM)-Signals repräsentiert.

16. Verfahren, wie in einem der Ansprüche 9 bis 15 beansprucht, **gekennzeichnet durch** Anzeigen eines gemessenen Signals der Größe zum Vergleich mit der genannten grafischen Repräsentation.

17. Verfahren, wie in Anspruch 16 beansprucht, **gekennzeichnet durch** Vergleichen der grafischen Repräsentation mit dem genannten Signal **durch** Überlagern der genannten grafischen Repräsentation dem genannten angezeigten Signal.

18. Verfahren, wie in Anspruch 17 beansprucht, **dadurch gekennzeichnet, dass** der genannte Überlagerungsschritt ein automatisches Einstellen der genannten grafischen Repräsentation auf den Maßstab des genannten gemessenen Signals umfasst.

19. Verfahren, wie in einem der Ansprüche 16 bis 18 beansprucht, **dadurch gekennzeichnet, dass** das genannte angezeigte gemessene Signal kontinuierlich über der Zeit variiert.

20. Verfahren, wie in Anspruch 18 beansprucht, **gekennzeichnet durch** Anzeigen von Offset- und Hervorheben von Signal-Abschnitten, die von in verschiedenen Zeitperioden gemessenen Signalwerten abgeleitet sind.

21. Computerprogrammerzeugnis, **gekennzeichnet durch** einen computerlesbaren Code, der, geladen in eine digitale Verarbeitungsvorrichtung, die Ausführung des Verfahrens veranlasst, wie es in einem der Ansprüche 9 bis 18 beansprucht ist.

22. Computerlesbares Speichermedium, welches das Computerprogrammerzeugnis, wie es in Anspruch 21 beansprucht ist, trägt.

## Revendications

1. Système de contrôle et de commande du fonctionnement d'un dispositif médical implantable, comprenant
des moyens (26, 10) de communication avec le dispositif (10) médical,
des moyens (22, 24) d'affichage d'une représentation graphique sous la forme d'une courbe ou d'une forme d'onde (400) d'une quantité influencée par le fonctionnement du dispositif médical,
des moyens (22) d'association de paramètres relatifs à la commande du dispositif à des parties de la représentation graphique,
des moyens (32, 22) de sélection et de déplacement de points de la représentation graphique pour modifier ainsi la forme de la représentation graphique affichée afin d'illustrer un effet fonctionnel souhaité du dispositif médical sur la quantité, et
- des moyens (22, 24), sensibles à la forme modifiée, de modification des valeurs des paramètres associés en fonction de la modification des parties.

2. Système suivant la revendication 1, **caractérisé en ce que** la quantité est au moins une activité physiologique influencée par le dispositif médical.

3. Système suivant la revendication 1 ou 2, **caractérisé par** des moyens de fixation d'une valeur de paramètre modifiée autorisée la plus proche en réponse à la forme modifiée de la représentation graphique.

4. Système suivant l'une quelconque des revendications 1 à 3, **caractérisé en ce que** la forme d'onde est une représentation de la variation de la quantité en fonction du temps.

5. Système suivant l'une quelconque des revendications précédentes, **caractérisé par** des moyens (26, 10) de recueil de données se rapportant à la quantité et des moyens (22) de construction d'une représentation graphique de la quantité pour un affichage.

6. Système suivant l'une quelconque des revendications précédentes, **caractérisé en ce que** plusieurs paramètres sont associés à des parties de la même représentation graphique de manière à ce que les moyens sensibles à la forme modifiée d'une représentation graphique soient conçus pour modifier les valeurs des plusieurs paramètres en fonction des parties modifiées.

7. Système suivant l'une quelconque des revendications précédentes, **caractérisé en ce que** la représentation graphique est une forme d'onde représentant l'activité électrique d'un système cardiovasculaire d'un patient.

8. Système suivant l'une quelconque des revendications précédentes, dans lequel le dispositif médical est un dispositif de stimulation cardiaque.

9. Procédé de contrôle et/ou de programmation du fonctionnement d'un dispositif médical implantable, **caractérisé en ce que** :
- on affiche une représentation graphique, sous la forme d'une courbe ou d'une forme d'onde (400), d'une quantité influencée par le fonctionnement du dispositif médical,
- on projette des paramètres relatifs à la commande du dispositif sur des parties sélectionnées de la représentation graphique,
- on modifie la forme de la représentation graphique pour afficher un effet fonctionnel souhaité du dispositif médical sur la quantité , et
- on programme le paramètre de commande associé en réponse à la forme modifiée.

10. Procédé suivant la revendication 9, **caractérisé en ce que** la quantité est au moins une activité physiologique.

11. Procédé suivant la revendication 9 ou 12, **caractérisé en ce que** le stade de projection, comprend la projection d'amplitudes de paramètres de dimension sélectionnées de la représentation graphique et le stade de modification comprend la modification de l'amplitude du paramètre de commande en réponse à un changement des dimensions.

12. Procédé suivant l'une quelconque des revendications 9 à 11, **caractérisé en ce que** la représentation graphique est une forme d'onde représentant la variation de la quantité en fonction du temps.

13. Procédé suivant l'une quelconque des revendications 9 à 12, **caractérisé par** la production de la représentation graphique à partir d'une pluralité de courbes et/ou de valeurs qui sont mesurées.

14. Procédé suivant l'une quelconque des revendications 9 à 13, **caractérisé en ce que** l'on projette plusieurs paramètres sur une représentation graphique et on modifie les plusieurs paramètres en réponse à la modification de la courbe de la représentation graphique.

15. Procédé suivant l'une quelconque des revendications 9 à 14, **caractérisé en ce que** le stade d'affichage comprend l'affichage d'une courbe représentant au moins un cycle d'un signal d'électrocardiogramme (ECG) ou d'électrogramme intracardiaque (IEGM).

16. Procédé suivant l'une quelconque des revendications 9 à 15, **caractérisé en ce que** l'on affiche un signal mesuré de la quantité pour comparaison à la représentation graphique.

17. Procédé suivant la revendication 16, **caractérisé en ce que** l'on compare la représentation graphique au signal en superposant la représentation graphique sur le signal affiché.

18. Procédé suivant la revendication 17, **caractérisé en ce que** le stade de superposition comprend l'ajustement automatique de la représentation graphique à l'échelle du signal mesuré.

19. Procédé suivant l'une quelconque des revendications 16 à 18, **caractérisé en ce que** le signal mesuré qui est affiché varie continuellement en fonction du temps.

20. Procédé suivant la revendication 18, **caractérisé en ce que** l'on affiche des parties de signal de décalage et de contraste provenant de valeurs du signal mesurées dans des laps de temps différents.

21. Produit de programme informatique, **caractérisé par** un code pouvant être lu par un ordinateur qui, lorsqu'il est chargé dans des moyens de traitement numérique, provoque l'exécution du procédé tel que revendiqué, suivant l'une quelconque des revendications 9 à 18.

22. Support de mémorisation pouvant être lu par un ordinateur, comportant un produit de programme informatique tel que revendiqué à la revendication 21.
